# EUROPEAN PATENT APPLICATION

(11) **EP 3 967 148 A1**
(43) Date of publication of application: **16.03.2022**
(21) Application number: 19927585.0
(22) Date of filing: 06.05.2019
(51) Int. Cl.: A23G 3/48, A23G 3/36

(54) **EYE-NOURISHING AND EYESIGHT-IMPROVING HERBAL CANDY CONTAINING NUTRIENTS FOR HUMAN EYE LENS AND RETINAL PHOTOSENSITIVE CELLS**

(71) Applicant: Shenzhen Linpingxijing Vision Technology Co., Ltd, Shenzhen, Guangdong 518000 (CN)
(72) Inventor: Gao, Si, Shenzhen, Guangdong 518029 (CN)
(74) Representative: Wilson Gunn
(86) International application number: PCT/CN2019/000089
(87) International publication number: WO 2020/223832

(57) **Abstract**

An herbal confectionery candy for eyesight improving is provided by the present invention and belongs to the technology field of eye vision nutrient health care and Chinese herbal medicine conditioning and eye rehabilitation. The herbal confectionery candy for eyesight improving serves as a supplemental nutrient for human eye lens and retinal photoreceptor cells and provides Chinese herbal medicine eye conditioning, preventive health caring and repairing effects. It contains 12 of the most directly effective nutritional ingredients coupled with 54 kinds of Chinese herbs, processed by the modern technologybased functional candy industry. It is easy to carry around and easy for oral intake. It features the functions of eliminating the intraocular free radicals, repairing damage to eyesight caused by blue light and tonifying eyes with nutrients.

## Description

### TECHNICAL FIELD

The present invention relates to a supplemental nutrient for human eye lens and retinal photoreceptor cells, provides Chinese herbal medicine eye conditioning, preventive health caring and repairing effects, which belongs to the technology field of eye vision nutrient health care and Chinese herbal medicine conditioning and eye rehabilitation; The present invention particularly relates to an new achievement confirmed by the international nutriology as an essential nutrients for eye lens and retinal photoreceptor cells, and the bio-antioxidants for intraocular cells and tissues, supplemented with effective Chinese medicine drugs have been verified by Chinese medicine practitioners in the past thousands years, processed by the modern technology-based functional candy industry, provides people a carry-on diet therapy for eye caring, preventing eye diseases, eliminating intraocular free radicals eliminating, repairing and reducing the accumulation of electronic blue light damage, serves as an ophthalmic nutrition conditioning functional candy.

### The evolution of the eye:

The oldest life form-microbes were born on Earth about 3.95 billion years ago. Cyanobacteria appeared about 3.5 billion years ago, about 2,000 species of cyanobacteria are known, which are widely distributed around the world. Cyanobacteria contains chlorophyll a, which is the earliest oxygen-evolving photosynthetic organism. Cyanobacteria played an important role in the change the anaerobic atmosphere to the aerobic environment. Without oxygen, the life body can only be as small as bacteria; some cyanobacteria can directly fix the nitrogen in the atmosphere, and the protein appears later; with more oxygen, the organism can store more energy, the food chain becomes longer, and the predation evolves into the most efficient way of life. The Cambrian began about 541 million years ago, in the preceding tens of millions of years, the organisms have formed complex and moderate populations in the ocean: some are suspended feeders, some are filter feeders, some passively absorb the nutrients, and some can even move. Predation promotes the survival competition and evolution between biological populations, and gradually develops sense of touch, taste, smell, and auditory, prey food from near to far away, and escape from being preyed. Survival or extinction forces predators and prey to accelerate their own evolution, and individuals develop toward large-size.

Scientists discovered the oldest eye on the trilobite fossils of the early Cambrian period about 530 million years ago. The ancient trilobite was considered as the first animal evolved the original eye on Earth; the fossil was found in the northern Estonian. The ancient trilobite has original compound eyes, which is simpler than the compound eyes of crabs, bees, flies and other insects and crustaceans, the compound eye is composed of several tiny visual organs "ommatidia", which is not a complete monocular. The trilobite compound eyes consist of about 100 ommatidia. Compared with the current animal compound eyes, these ommatidia are more sparsely distributed, simpler in structure and lower in resolution than monocular; the trilobite has no soft lens in the ommatidia, but has lenses made of transparent calcite. Evolutionary biologists calculated that it would take about 400,000 years to evolve from the original photoreceptor cells that formed the retina 540 million years ago to the original compound eye. Compared to the sense of touch, taste, smell, and auditory, the advantages of the eye are very obvious, vision can provide more environmental information to the organism. Animals with eyes can perceive more complex environmental information, have more abundant food and lives; without eyes, the animals are difficult to forage and are easily hunted and extinct. The formation of the eye changed the process of biological evolution on the earth, completely subverted the rules of life activities, a world full of brutal competition began, many animals became active predators, which is the cause of the Cambrian explosion, when a creature has eyes, forces other creatures to evolve eyes to chase prey or avoid being hunted; this has intensified the evolutionary competition of species, more and more complex new species have evolved. The appearance of the eyes promotes the birth of complex living species, it helps the life of the earth lived in darkness for 3.4 billion years evolved into unprecedented new species with sudden prosperity. What a great and lengthy evolutionary miracle! There will be no human beings and progression of evolution without the appearance of eyes!

The octopus appeared on Earth about 270 million years ago. The octopus has well-developed eyes, has camera-like structure, which is the only similarity between octopus and humans. The cephalopods have big eyes, and part of the brain processes visual information and uses this information to control their camouflage. There are no obvious difference between the structure of the cephalopod eye and the human eye; in the "eye" with which the Loligo perceiving the temperature, the photonic chemical reactions happens, like the chemical reactions in the human retina or photographic film, the electromagnetic wave including light and infrared rays absorbed by the photoreceptor cells trigger the combination of photosensitive molecules(or temperature sensitive molecules in the squid, to escape of predation from whale the squid dives into the dark sea), and the photosensitive molecules acts on the optic nerve and transfers to the brain to form the image of the object. The cornea of the octopus eye does not form a single entire piece, while has a hole in the center. In human, the focal length adjustment of the eye to objects at different distances is accomplished by changing the curvature of the lens, while in the octopus, by moving the lens away or close to the retina, like the camera rotates the lens. The octopus eyelids are also different from the human eye, the octopus eyelids have a ring-shaped muscle, which can cover the eyes quickly like a camera lens shutter when the eyes are closed. No animal in the ocean has the same sensitive eyes as octopus and its relatives. Only owls, cats, and human have the similar eyes. There are about 63,000 cone cells per square millimeter in the octopus retina, 105,000 in squid, 162,000 in Loligo; 16,000 in spider (non-insect); 50,000 in carp; 397,000 in cat, 400,000 in human and 680,000 in owls. In the animal world, giraffes can see 8 kilometers away, the eagle has the best eyesight, which can see 36 kilometers away. See more far indicates that the high-density cone photoreceptor cells in the retina and have high resolution. There for, although the human eye is more precise and more complex, it still has limitations of adaptation.

Ancient anthropologists studied and found that hominoids living in forested are not the ancestors of modern humans, but the ancestors of monkeys, gibbons and orangutans. The earliest ancestors of modern humans were australopithecines lived in southern Africa about 5 million years ago, and the physical structure characteristic of the australopithecine fossil showed that they were able to walk upright and were able to use natural tools. Australopithecus afarensis is a subtribe of australopithecine, with averaged heights of 1.2-1.3 meters and weighed about 25 kilograms, the average brain volume is less than 450 milliliters. The A. afarensis has flexible bodies and hands, they can not only use natural tools, some "smart one" also can make simple tools. They live in groups in dry, open grassland, collect young leaves and fruits by cooperation, catch turtles, snakes and small mammals frequently, and occasionally hunt large mammals. About 3 million years ago, during the long-term struggle and fight with nature, the australopithecines not only formed the simple and original language in labor, but also learned how to use stone to strike together to make simple tools and finally became the earliest human. The eye is the most important organ in human, about 80% of the knowledge and memory are acquired through the eyes. Lived in the dry, open grassland for millions of years, they used the eyes when collecting, capturing, hunting, avoiding enemies and using tools in labor. Human eye vision is suitable for observation of targets from 50cm to long distances.

Human visual troubles: machine printing has made the use of fine and dense text books widely popular, and reading and Clerical work has become a growing job. Read fine printed characters in 50cm for a long time, excessive accommodation of the lens leads to eye strain and focus adjustment dysfunction, which is the main cause of myopia; thoroughly changed the human eye environment in the past millions of years of field work, resulting in more and more reading and Clerical workers develop myopia, astigmatism or amblyopia. With the changing of the environment, the biological organs can adapt to the new environment by micro-evolution, which needs heritable gene mutation and stable expression in the offspring, to be final adaptation need to accumulate in lots of generations in tens of thousands of years. It is extremely difficult to keep up with the need for dramatically environmental changes in the past few decades, and this this is the fundamental cause of large number of people developed myopia after Industrial Revolution, while the incorrect reading posture or poor lighting is a secondary one.

A small number of professionals Watching computer workers all day, more and more students study by learning machines or computers; smartphones are widely spread since been invented, there are abundant attractive contents in the phones, which have rapidly changed the lifestyle of people in the daily life such as private Contact, social communication, watching news, reading books, watching film and television, shopping, payment, checking the account, traffic navigation, finding location. the people are using phones while they are in leisure time, working, taking bus, walking, at home, eating, before or after sleeping. More and more people are addict to electronic products. According to survey from CCTV reported that people watch mobile phones for 5 to 10 hours a day. The physical nature of light is the high frequency electromagnetic waves. The fluorescent screen emits electronic bright light, contains the blue light with wavelength between 400nm and 500nm which is close to ultraviolet light. The shorter the wavelength, the higher the energy, it can easily penetrate the lens, generate free radicals which can cause opacity to the lens; when the blue light reaches the retina, it can induce photochemical damage to the photoreceptor cells, the retina contains 130 million rod cells and 7 million cone cells, the accumulated damage can cause photoreceptor cells apoptosis gradually, which is different from the myopia caused by the dysfunction of lens accommodation, eventually cause blurred vision and reduce of resolution of the macular (completely composed of compact cone cells), you have to pull the object closer to see clearly. When the blurred vision keeps aggravating, the people will be quasi-blind, the reason is not simple as the myopia caused by accommodation dysfunction, which has no decrease of retina resolution. After years of accumulation of photochemical damage caused by screen emitted lights, the visual acuity will decrease continuously until blindness, eventually, a large number of people will become blind in their later years, the photochemical damage caused by screen emitted lights are much severer than myopia!

The instruction of "Protect children's eyesight and bringing them a bright future" pointed out that the high incidence of myopia among students, which affects more and more children at a younger age and undermines their health, is a major problem concerning the future of the country and the Chinese nation, this have to be changed. The related departments should roll out an effective preventive scheme, and all the society must take actions to protect the eyesight for the kids and bring them a bright future. According to "the instruction of protect the children's eyesight triggered hot discussion in Xinjiang", Xiaojing Zhou, one of the parent of children from the Second Primary School in Aksu city, said that the children learned how to use smartphone from their parents who always keeping playing with their mobile phones. Studies showed that the lens of children are more transparent than adults, which the blue lights can easily pass through and 85% lights reach the retina. the eyes are most vulnerable to blue lights in adolescence, the extent of blue light caused damage differs by age:80-90% for 6-19 years old, 60-70% for 20-39 years old, and 50% for 40-60 years old. Without any intervention, most of the children born in this century will have lower eyesight and retina resolution than all their predecessors, and will not be able to take jobs that require excellent vision! This will be a huge danger for the country and the nation!

Blu-ray damage mechanism: Eye care Association of WHO announced that from 2006 to 2008, due to blue light radiation, more than 30,000 people worldwide were blinded each year; and an orange warning was issued at the end of 2009: "Blue light radiation, a potential hidden threat to humans It will far exceed the destructiveness of Sudan Red, melamine, SASI, HINI avian flu, etc., invisibly devour human eyes." According to the data of the Ophthalmology Branch of Chinese Medical Association, 63.5% of the 420 million netizens in China have different extents of eye diseases due to blue light radiation, including decreased vision, cataract, and blindness. In daily life, Blue light is abundantly contained in lights emitted from energy-saving LED lights, computer monitors, smart phones, digital products, LCD screens, and bath heaters etc.

Why eye strain, dry eye, irritation symptoms are prone to occur when using LED lamps, computers, smartphones, etc. for a long time? Most of the LED lights use phosphors to emit white light which is excited by the blue light of 450-455 nm wavelength. The shorter the wavelength, the stronger the excitation ability. The factory setting of light wavelength of the LED lamp is within 450-460nm, which can induce strongest radiation damage. Change the wavelength will reduce the ability of exciting the phosphors and affecting the luminous efficiency decreases. To improve brightness, the factory will strengthen the blue light intensity of the LED light source. If the human eye uses such a light source for a long time, they will be damaged by blue light. In addition, the longer the LED lamp uses, the faster the phosphors degrade, and the blue light that is not converted by the phosphor is directly emitted which enhancing eye damage. Long time use of LED light will easily cause dizziness and uncomfortable, which will damage your eyes and increase the chance of eye disease. LED lights are only suitable for short-term use as an auxiliary light.

German ophthalmologist pointed out that when inappropriate light is continuously injected into the eyes, it will cause dysfunction, especially the short-wave blue light emitted by tricolor lamp, computer screens, smartphones. Short-wave blue light has extremely high energy and can penetrate the lens and reach the retina, causing photochemical damage to the retina, leading to cell damage in the macular. Blue light can increase the amount of toxin in the macular and aggravate the macular disease, which seriously threatens the health of the retinal fundus. Exposure to high-intensity blue light for a long time doubles the incidence of macular degeneration.

Blue light can cause blurred vision, induce visual fatigue and video display terminals(VDT) syndrome. After the blue light enters into the eye, it focuses in front of, instead of on the retina due to the wavelength difference, this increases the distance of focus point of different colors in the eye, which is the main reason for the blurry vision. The blue light injection can aggravate the color difference and the visual blur, induce hypertonia of eye muscles, increase the blood supply to the eyes, thereby aggravate visual fatigue. Blue light can cause glare: the blue wavelength is between 400nm and 500nm, has the shortest wavelength and highest energy; the short-wavelength lights scatter more easily when they small strike particles in the air, which is the main cause of glare induced by blue light.

Melatonin is one of the influencing factors of the human body biological clock, which is secreted by the pineal gland in brain; under the illumination of 1.3 lux of blue light at noon, melatonin secretion is terminated. The intensity of blue light emitted by LED energy-saving lamps, computer screens and mobile phones is close to the intensity of blue light in sunlight at noon, but closer to the eyes, at this time, the damage of blue light to the retina and the influence to the secretion of melatonin has far exceeded the intensity induced by 1.3 lux blue light; the blue light can stimulate the brain, inhibit melatonin secretion, and increase the secretion of adrenocortical hormone, thereby destroying the balance of hormone secretion and directly affect sleep quality. The research from Harvard University shown that use a computer or a smart phone before going to sleep, the blue light they emit is enough to stimulate the brain, keep the brain in a state of excitement, affect the normal biological clock, take longer time to fall asleep, easier to wake up and other symptoms of insomnia. The human body begins to secrete large amounts of melatonin at 9 or 10 o'clock in the evening to cause drowsiness, but due to blue light, the melatonin secretion will be greatly reduced. Besides, Melatonin is very important for the immune system and anticancer function. Don't sleep well means that all organ systems are prone to dysfunction and increase the incidence of diseases.

Visual Science: Human are not born with 20/20 vision, but they develop normal vision after the early months of pregnancy and postnatal 13 years. Except for the influence of the external environment on visual formation, the supply of eyes needed nutrients during development is crucial for the development of healthy and normal eye tissue. In the third week of pregnancy, the eyes begin to develop. The fetal development is closely related to nutritional status of pregnant women, the fetal eye development is determined by the concentration of carotenoids such as lutein in the body of pregnant women. Lutein can prevent myopia, deficiency of nutrients such as lutein is one of the reason that myopia can be inherited, that the fetus cannot get enough lutein from the mother during development, causing the eye to excessive accommodation to the light and induce myopia. The breast milk contains Lutein, the fetus can still get Lutein needed for eye development thought breastfeeding after birth. Children's myopia, hyperopia, astigmatism are manifestations of visual development retardation, if the nutrients needed for eye development are not supplemented in time, it may form amblyopia and increase the difficulty of visual recovery. After light enters the eye, the light passes through the cornea, aqueous humor, lens, vitreous, and reaches retina, there are two kinds of photoreceptor cells in the retina: rod cells and cone cells; rod cells are responsible for dim light vision, and cone cells are responsible for processing colors and details; when exposed to light, a series of chemical reactions occur and activate the rhodopsin which can produce electronic pulse signal in the optic nerve.

As mentioned above, when high-energy contained blue light is injected into the eye, greater amounts of free radicals are generated in the lens and retina. Long-term research by life scientists has revealed that the oxidative damage of reactive oxygen and free radicals in cells and tissues in the human body is the main causes of aging and other more than 100 diseases, including arteriosclerosis, hypertension, thrombosis, coronary heart disease, cerebral infarction, cancer formation and metastasis, pancreatitis, diabetes, kidney disease, liver disease, gastrointestinal ulcers, vision deterioration, macular degeneration, cataract, hyperthyroidism, skin aging, Alzheimer's disease, epilepsy, etc. Each cell of a human body is under attack by 1-10 thousand times per day from free radicals. According to the Mutation-Accumulation Theory: lesions and senescence start from each single cell, aging occurs under each breath; when cell and tissue is exposed to reactive oxygen or free radicals, the oxidative stress can cause various oxidation reactions of cell components including lipids, glucose, proteins, DNA, and RNA, induce oxidative damage such as denaturation, cross-linking, and breakage, which in turn leads to destruction of cell structure and function, as well as damage to the tissues and organs. In order to maintain the body's normal operation and health, to resist the damage of various reactive oxygen species and free radicals: 1.there are various antioxidants in the body to inhibit the production of various reactive oxygen species and free radicals; 2.capture and combine the formed active oxygen and free radicals to make it a stabilizer without aggression; 3.repair or regenerate the cell damaged by free radicals; 4.to perfect the defense ability, Under certain conditions, follow-up support by various antioxidant enzymes like a reserve force. Although there are various antioxidants in the aerobic organisms that defense the reactive oxygen species, when this defense effect is insufficient, such as ultraviolet light and high-energy blue light irradiation, disease, inflammation, ischemia, antibiotics, anesthetics, aging, various chemical pollution, moldy food, smoking, strenuous exercise, mental stress, the balance between the production and elimination of reactive oxygen species and free radicals will be destroyed, and oxidative damage occurs.

Studies on the macular and lens have shown that only lutein and zeaxanthin are present in these two important tissues, while no other carotenoids are detected. Therefore, modern research on the eyes focuses on lutein and zeaxanthin. Lutein is one of the carotenoids, has 8 different isomers, most of which are mainly all-trans, Lutein can hardly be artificially synthesized, until now, it only can be extracted from plants, Marigold is the main raw material source for extraction of Lutein. Lutein has several physiological functions, lutein is a potent antioxidant and a ultraviolet absorber, can reduce the oxidative stress induced damage on the eye, resistant to ultraviolet-induced oxidation in the macular area; lutein and zeaxanthin can selectively accumulate in the macular area, preventing the damage of the singlet oxygen produced by ultraviolet to the macular, they suppress the oxidative degeneration of polyunsaturated fatty acids in retinal cells by quenching singlet oxygen to inhibit free radical formation; When there are enough lutein and zeaxanthin in the macular, it can absorb ultraviolet rays to prevent oxidative damage of the macula, protect the macula from damage, and prevent blindness causing eye diseases such as age-related macular degeneration and cataract. Zeaxanthin and lutein are always existing together with similar effects. The deficiency of them may cause macular degeneration and blurred vision, and further induce myopia syndrome, cataract and other eye diseases. Oxidation of proteins in the lens plays an important role in the formation of cataracts, while zeaxanthin and lutein can prevent the oxidation of proteins and lipids in the lens, thereby reducing the incidence of senile cataract. According to statistics in USA, the leading causes for the decline in visual acuity of adults over 40 years old is age-related macular degeneration(AMD) and senile corneal opacity, which affect 25 to 30 million people worldwide; A survey of 500 cataract patients in Japan found that diets rich in lutein and zeaxanthin have a significant preventive effect on cataract; The US Food and Drug Administration(FDA) has recommended that the aged people over 65 years old should take daily oral supplementation of lutein and zeaxanthin 6mg per day to prevent the deterioration of vision avoid developing into blindness. While, still lacking popularization in China.

Anthocyanins extracted from blueberries and bilberry can be used in ophthalmology for the treatment of night blindness and diabetic retinopathy. Rhodopsin is a light-sensitive protein in retinal photoreceptor cells, it will immediately photobleaches when rhodopsin is exposed to light reached the retina, and transmit the chemical change to the visual center of the brain to produce a visible image. Rhodopsin can be naturally re-synthesized in the dark, the anthocyanins extracted from blueberry and European bilberry can promote the re-synthesis of rhodopsin in the dark and improve the sensitivity of the retina to light. Anthocyanin can enhance night vision and reduce the dark adaption time under dim light; On the contrary, to the strong light induced short term poor vision, the cranberry extracts can shorten the vision recovery time. In World War II, the British Air Force bombed the German military industrial base Ruhr, but the lights are very dim due to the wartime blackout policy, to help the pilots and bombers can see the target at night, the military let them ate fresh blueberry jam to improve night vision. In Western Europe, there is a blueberry juice oral liquid on sale for preventing visual fatigue. In the United States, the recommended dose for intake of blueberry anthocyanins is 180-215 mg per day.

### BACKGROUND

The present invention is an integration and innovation based on collecting all the best scientific research achievements in each field, 5 existing technology are used for reference:
1. The developed countries take blueberry lutein as nutrient supplementation orally to prevent and treat age related macular degeneration and lens opacity;
2. In the 290 kinds of effective ophthalmic Chinese herbal medicine which have been verified by Chinese medicine practitioners in thousands of cases treatment in the past thousands of years, choose 54 best herbs after compared one by one;
3. The successful experience of functional candy production in China such as: mint cooling candy, eucalyptus oil throat candy, Sweets Golden Throat functional candy;
4. The production process technology and marketing accumulation of sandwich candy, liqueur-filled chocolate, etc. in china;
5. The capsule dosage form of Western medicine, the capsule shell material selection and processing method, the assembly technology of wrapping the medicine compound core; the present invention uses above 5 existing technology for reference, draws on the existing technologies of the five aspects, selects each of its excellent essence parts, comprehensively improves, integrates the innovatively invent an original and unique product.

### SUMMARY

The meeting of "Preventive Treatment of Disease" decided that from 2008 onwards, implement a health project of the "Preventive Treatment of Disease" in the country, give full paly to the function Traditional Chinese medicine in prevention, health care, preservation, protection, and rehabilitation area. " Take good care of children's eyes and bring them a bright future" pointed out that the high incidence of myopia among students, which affects more and more children at a younger age and undermines their health, is a major problem concerning the future of the country and the Chinese nation, this situation has to be changed, the related departments should roll out an effective preventive scheme, and all the society must take actions to protect the eyesight for the kids and bring them a bright future.

In response to the call of "Give full paly to the function Traditional Chinese medicine in prevention, health care, preservation, protection, and rehabilitation area" of the conference of "Preventive Treatment of Disease", and response to the instruction of "protect the eyesight of the kids" which pointed out "myopia is a major problem concerning the future of the country and the Chinese nation" , "all the society must take actions to protect the eyesight for the kids", and "roll out an effective preventive scheme"; the present invention broadly collects the latest scientific research results in the field of eye health care in the United States and Europe, combined with accumulated successful experience of Chinese traditional medicine in ophthalmology treatment practice, designed a supplemental nutrients of lens and retinal photoreceptor cells, serving as a functional candy for eye conditioning, eye health caring and injury repairing. The present invention belongs to the field of eye vision nutrient health care, Chinese herbal medicine conditioning, and eye rehabilitation technology; it has been confirmed by the New Achievement of International Nutriology as essential nutrients for eye lens and retinal photoreceptor cells, as well as the bio-antioxidants for intraocular cells and tissues. Specifically, this invention contains 12 of the most directly effective nutritional materials screened out from the past research results of the eye care scientists, supplementing with 54 out of 290 effective Chinese herbal medicine drugs which have been verified by Chinese medicine practitioners in thousands of cases of treatments in the past thousands of years. In addition of establishing its theoretical foundation on all the scientific research achievements in the field of eye health care, this invention is processed by the modern technology-based functional candy industry. It aims to provide people with a candy function as carry-on diet-based therapy for eye caring, vision sight improving, and preventive treatment, eliminating the intraocular free radicals while repairing the accumulation of electronic blue light damage induced eye conditions;

the object of the present invention: achieve a world leading original product invention patent, apply PCT international invention patent on the same day. After proved by initial evaluation report from PCT (success rate approved by the US and EU patent more than 90%), plan to finance and apply for patent protections of the G20 world's largest industrial countries, declare registered trademark protections of the world's 60 largest consumption countries. The population in all G20 members and 60 largest consumption countries accounted for more than 3/5 and 4/5 of the global population respectively, the invention will become an extensively demanded health care product for the people over addicted to playing on the cellphone, and the patients with various types of retinal degeneration and vision loss; The global industrial scale will reach 10 billion US dollars after 5 years of production. This is a super unicorn project that attract more attention from global financial investment community. After 10 years of production, the global industry scale can reach 1 trillion yuan. The present invention is an original invention of a new product by Chinese, it will be protected by intellectual property rights and stride to the world for the benefit of all mankind as an approved invention patent by G20 largest industrial countries and registered trademarks of the 60 largest consumption countries.

The present invention is applicable to the following populations: people who in their personal and work life spend long hours using computers and phones, LED light users, students with myopia symptom complex, elderly people with deteriorated vision, workers working in strong light, welders, workers exposed to direct sunlight, plateau residents exposed to strong radiation and freezing environment residents exposed to optical pollution. Based on consumers' different demands, the product offers four formulas, being A, B, C and D, to provide different effects. 130 examples of production are recorded in the patent description.

The list of raw materials accurately and preferentially selected in various fields of ophthalmology, and the creative production process of present invention is as follows:

### 1. Human lens and retinal photoreceptor nutrients, sorted according to importance (select 5 or more to join the actual production group):

(1). Rhodopsin nucleotides 0.0002 to 0.001 parts; Rhodopsin is a key photosensitive pigment in the retinal photoreceptor cells, it can be decomposed to form activated rhodopsin when exposed to light, which generate an electronic pulse, The function of rhodopsin is to convert the received light stimulus into pulse signal of the optic nerve and transmit to the brain for formation of vision; the light sensitivity of the photoreceptor cells is closely related to the stock of the undecomposed photosensitive pigment in the cell, with the age increases, the photosensitive pigment decomposition is accelerated; even the slightly decrease of photosensitive pigment concentration can greatly reduce the light sensitivity of the photoreceptor cell, When the rhodopsin is decomposed by 0.50% in the human retina, the photosensitivity of the rod cells is reduced by 2000 times; so the photosensitivity of the retina is closely related to the ratio of synthesis and decomposition of rhodopsin, particularly to the recovery of rhodopsin during dark adaption.
(2). 11-cis-retinal: 0.0002 ~ 0.001 parts; retinal also known as vitamin A aldehyde, chemical formula: C₂₀H₂₈O, 11-cis retinal is the most important isomer in the 5 isomers, which can be found both in the rod and the cone cells; 11-cis retinal is the prosthetic group of rhodopsin, which forms rhodopsin together with the opsin; retinal is important signal transduction molecule in the development of the eye, 11-cis retinal absorbs light instantaneously (this reaction takes only 1 trillion seconds) isomerize to all-trans retinal, which leads to conformational change of rhodopsin and initiates nerve impulses transmitted to the brain and forms vision; in the process of decomposition and re-synthesis cycle, some of the retinal consumed.
(3). Lutein ester 0.001 ~ 0.006 parts; Lutein has important protective effect on the macula of the retina, deficiency of lutein can cause macular degeneration and blurred vision, thereby impair vision and induce myopia and other symptoms; the main physiological functions of lutein on the eye is as an antioxidant and photo-protective effect. The optic nerve is non-renewable and extremely vulnerable to harmful free radicals. Lutein can inhibit the formation of harmful free radicals due to its anti-oxidation effect; lutein can absorb a large amount of blue light, which has the similar wavelength to ultraviolet light, the blue light is the most harmful visible light reaches the retina. Before reaching the photoreceptor cells of the retina, the light first passes through the area with highest concentration of lutein, if the lutein content in the macular area of the retina is enough, the damage can be minimized.
(4). Zeaxanthin 0.001 ~ 0.005 parts; Zeaxanthin is one of the two carotenoids in the human lens, as a strong antioxidant, it can quench the triplet state of singlet oxygen and photosensitizer, remove oxygen free radicals, prevent membrane lipid peroxidation, reduce lipofuscin formation, prevent cataract formation; people with high intake of lutein and zeaxanthin may reduce cataract by 19% to 22%; at the center of the macular region, perceive the most intensive incident light and produces large amounts of reactive oxygen; lots of studies have shown that zeaxanthin specifically absorbs the blue light which is most damaging to the retina, thereby protecting the cones in the fovea; studies have shown that short-term increase the intake of zeaxanthin can increase the macular pigmentation, thereby enhance the ability of the macula to resist harmful substances and light, prevent and delay the age-related macular degeneration.
(5). Vaccinium myrtillus anthocyanin 0.03~0.15 parts; European bilberry is often used to improve night vision. During World War II, the British Air Force pilots used European bilberry jam to improve night vision and execute night flight mission better. Studies have shown that supplement of European bilberry can inhibit or even reverse the ophthalmological diseases of macular degeneration; its anthocyanins can relief eye fatigue, improve night vision, prevent cataract, improve amblyopia, accelerate the rhodopsin regeneration rate, improve dark vision, reduce dizziness, improve dark adaptation ability, improve night blindness, improve eye fatigue recovery; protect retinal capillary, improve eye blood circulation, improve vision.
(6). Blueberry anthocyanin 0.04~0.22 parts; Blueberry anthocyanin has a good maintenance effect on the eyes, it is a star that awakens the eyes function: it can relieve eye fatigue, improve eyesight, prevent cataract and age-related macular degeneration. Clinical reports shown that it can promote the synthesis and regeneration of rhodopsin in photoreceptor cells of the retina, prevent severe myopia and retinal detachment, improve retinitis pigmentosa and amblyopia, prevent vision loss, improve eyesight; It can also strengthen the microvascular wall of the eye, promote the blood circulation of the micro vessels, maintain normal eye pressure, effectively inhibit the enzymes that damage the eye cells, relieve various eye diseases; it can enhance the visual acuity at night and improve the vision in dim light. Anthocyanin is helpful for drivers, especially those who drive at night, and those who watch the screen for a long time.
(7). β-Carotene 0.001 ~ 0.006 parts; the molecular structure of β-Carotene is equivalent to double vitamin A. After oral administration, 50% β-Carotene can be converted into vitamin A by the enzymes in the small intestine mucosa and liver, which has the effects of liver nourishing and eyesight improving, it can be used to treat night blindness. Epidemiological investigation shown that people who eaten carotene-rich food had significantly lower incidence of degenerative eye disease than those people who taken less carotene. The eyesight depends on the fundus macular, if lacking protection and support from enough β-carotene, the macular will have degenerative disorders, that is, aging, vision will decline and even night blindness occurs. These changes are more common in the elderly people, which have been considered as aging phenomenon by the medical researchers, they pointed out that this degenerative eye disease can be prevented by supplementing of β-carotene. In the retina, retinal rod cells are responsible for night vision by its component rhodopsin, β-carrot can promote rhodopsin to maintain its normal concentration, avoid dark adaptation prolongation induce by vitamin A deficiency, and avoid the photic injury after dark adaptation, β-carrot can also prevent night blindness, dry eye, corneal ulcer and keratomalacia. The people who often use the electronic screen should take β-carotene orally to protect against eye damage.
(8). Vitamin B2 0.0004 ~ 0.002 parts; Vitamin B2, also known as riboflavin, has two active forms: flavin adenine dinucleotide (FAD) and flavin mononucleotide (FMN) in the human body, which is involved in in the redox reactions. Recently studies have shown that riboflavin is also closely related to the antioxidant defense system in the body; the functions of vitamin B2 including maintaining the normal metabolism of the retina and cornea, improving vision, relieving eye fatigue; deficiency of vitamin B2 can cause the congestion, red eye, itchy, easy tears, lassitude, dizziness; the early manifestations of riboflavin deficiency are lassitude, hypodynamia, eyes itching, burning sensation, spasm, conjunctivitis, corneal neovascularization, etc.
(9). Docosahexaenoic acid powder 10% (DHA) 0.04~0.22 parts; DHA is an important component of brain cell membrane and retina, which accounts for 20% in the cerebral cortex and 50% in the retina. It plays an important role in promoting fetal retinal photoreceptor cell maturation. In the cerebral cortex, DHA is not only the main component of nerve conduction cells, but also the main component of cell membrane formation, it also plays important role in the axonal extension of nerve cells and the synapse formation, and maintain normal physiological activities of nerve cells. Elderly people take DHA-containing health products frequently can restore the degraded brain function and memory, it can be used for brain-strengthening and brain-nourishing; DHA can also enhance retinal reflex ability, increase the correct identify rate for luminance, improve visual function. From 1999 to 2000, the Retina Foundation of the Southwest conducted a 4-month feeding trial on infants, group one set as control group with normal diet, group two supplement with 0.35% DHA, and the third group supplement with 0.36% DHA plus 0.72% ARA; all the infants underwent scanning visual acuity (SVEP) visual acuity test 12 months later, the visual acuity in DHA+ARA group (promote optic nerve development) was significantly higher than control group.
(10). Vitamin C 0.06 ~ 0.3 parts; Vitamin C is one of the main components of the eye lens, it can reduce the damage of light and oxygen. Deficiency of vitamin C is prone to induce lens opacity and cause cataract. Vitamin C has many clinical applications in ophthalmology: it can reduce capillary fragility and permeability, prevent and treat hemorrhagic eye disease, capillary dysfunction, etc.; vitamin C can remove free radicals and antioxidant, participate in the redox metabolism in vivo, it can also prevent senile cataract, senile Macular degeneration and retinal degenerative diseases. American scientists have found that vitamin C has a special effect on the eyes and brain: scientists at the University of Oregon Health and Science (OHSU) found that the retina needs to be "bathed" in an environment with higher concentration of vitamin C to function properly.
(11). Haematococcus Pluvialis 0.0-0.3 parts; Human aging is mainly caused by free radicals induced oxidative damage. Haematococcus pluvialis is currently the best natural astaxanthin, which has good anti-oxidation effect, it can easily pass through the blood-brain barrier and cell membrane, directly remove intracellular free radicals, enhance cell regeneration ability, protect cell and DNA health, reduce oxidative debris accumulation in cell, thus delay cell senescence. The human retina and central nervous system (brain) are rich in unsaturated fatty acids, which can be easily oxidized by oxidation produced free radicals and causes peroxidative damage. The eye is the most vulnerable organ to oxidative damage, after affected, eye fatigue and decreased vision may occur, even presbyopia, cataract, glaucoma, blindness, retinopathy and macular degeneration etc. could happen in severe cases. Astaxanthin can help repairing oxidative damage in the eye, the Haematococcus Pluvialis extracts can cross the "blood-retinal barrier", reach any part of the eye, effectively prevent retinal oxidation and photoreceptor cell damage, repair oxidative damage cells. Particularly, astaxanthin has significant effect on treating retinal macular degeneration.
(12). Blueberry powder 0.2 to 1 part; Blueberry is rich in a variety of vitamins, minerals and fiber elements and other nutrients, its main ingredient is anthocyanin. Eat blueberries frequently can reduce fatigue of the eyes, increase the collagen in the eye, reduce intraocular pressure and promote the re-synthesis of rhodopsin, thereby improving eyesight, and make vision brighter. Therefore, people who have been engaged in long-term eye work, such as computer workers and editors etc. should properly supplement with a daily intake of blueberry to ensure that the eyes absorb enough blueberry anthocyanins. Anthocyanins have strong antioxidant effects, it can remove free radical, delay aging, prevent cell degeneration, in the meantime, anthocyanins can also reinforce Capillary elasticity and improve blood circulation.

The present invention provides an herbal confectionery candy for eyesight improving, wherein: the herbal confectionery candy comprises a first type of raw materials composed of human eye lens and retinal photoreceptor cells nutrients, comprising:
(1) rhodopsin nucleotides: 0.0002-0.001 parts; (2) 11-cis-retinal: 0.0002-0.001 parts; (3) lutein ester: 0.001-0.006 parts; (4) zeaxanthin: 0.001-0.005 parts; (5) accinium myrtillus anthocyanin: 0.03-0.15 parts; (6) blueberry anthocyanin: 0.04-0.22 parts; (7) β-carotene: 0.001-0.006 parts; (8) vitamin B2: 0.0004-0.002 parts; (9) docosahexaenoic acid: 0.04-0.22 parts; (10) vitamin C: 0.06-0.3 parts; (11) haematococcus pluvialis: 0.06-0.3 parts; (12) blueberry powder: 0.2-1 parts;
   more than 5 kinds can be selected from above 12 kinds of the human eye lens and retinal photoreceptor cells nutrients and added into an actual formulation group, and a total weight of selected raw materials should be between 0.3g and 1.8g;
   the actual formulation group comprises following groups: 11 kinds of the human eye lens and retinal photoreceptor cells nutrients are selected from (1) to (11) as group A, 10 kinds of the human eye lens and retinal photoreceptor cells nutrients are selected from (2) to (11) as group B, 8 kinds of the human eye lens and retinal photoreceptor cells nutrients:(3), (4), (6), (7), (8), (9), (10), (11) are selected as group C, 5 kinds of the human eye lens and retinal photoreceptor cells nutrients: (3), (7), (10), (11), (12) are selected as group D; the selected raw materials in all the groups must can be pressed into a flat oval shape candy core tablet with a designed size for use, and the flat oval shape candy core tablet should be able to be filled into a flat shell-shaped candy capsule to complete a confectionery product;
   the herbal confectionery candy for eyesight improving also comprises following component parts by a mass of a second type of the raw materials used against eye damage:
      (1) ginkgo leaves: 3-9 parts; (2) wood butterfly: 6-9 parts; (3) ligustrum fruit: 6-15 parts; (4) feather cockscomb seed: 3-15 parts; (5) mother of pearl: 10-30 parts; (6) dendrobium: 6-15 parts; more than 3 kinds can be selected from above 6 kinds of the raw materials used against eye damage and added into the actual formulation group, wherein, the (1) ginkgo leaves and the (2) wood butterfly must be selected as the raw materials of the actual formulation group;
   the herbal confectionery candy for eyesight improving also comprises following component parts by a mass of a third type of the raw materials used for the eyesight improving:
      (1) dream flower: 3-15 parts; (2) flos eriocauli: 9-12 parts; (3) equisetum: 3-10 parts; (4) goat liver: 30-60 parts; (5) semen thlaspi: 5-15 parts; (6) ghost eye: 6 parts; (7) chrysanthemum: 10-15 parts; (8) basil: 3-5 parts; (9) fructus rubi careani: 9-15 parts; (10) herba sceptridii: 6-12 parts; (11) raspberry: 5-10 parts; (12) semen allii fistulosi: 6-12 parts; (13) senecio: 15-30 parts; (14) plantago: 5-15 parts; (15) black soya bean flower: 3-9 parts; (16) vitis romanetii roman: 15-30 parts; (17) phyllanthus: 15-30 parts; (18) buckwheat leaves: 5-10 parts; (19) black bean: 5-10 parts; (20) raspberry root: 15-30 parts; more than an one kind can be selected from above 20 kinds of the raw materials for the eyesight improving and added into the actual formulation group;
   the herbal confectionery candy for eyesight improving also comprises following component parts by a mass of a fourth type of the raw materials used for liver clearing and the eyesight improving:
      (1) buddleia: 6-15 parts; (2) cassia: 6-30 parts; (3) abalone shell: 10-30 parts; (4) yemingsha: 3-10 parts; (5) sophora fruit: 5-15 parts; (6) glossy privet leaves: 10-15 parts; (7) herba blumeae megacephalae: 10-15 parts; (8) qinpi: 6-12 parts; (9) medlar leaf: fresh leaves 60-240 parts; (10) medlar: 5-15 parts; (11) prunella: 6-30 parts; (12) paper mulberry fruit: 6-10 parts; more than the one kind can be selected from above 12 kinds of the raw materials for the liver clearing and the eyesight improving and added into the actual formulation group;
   the herbal confectionery candy for eyesight improving also comprises following component parts by a mass of a fifth type of the raw materials used for liver nourishing and the eyesight improving:
      (1) rabbit liver: 30-60 parts; (2) nux prinsepiae: 3-10 parts; (3) pork liver: 60-150 parts; (4) carrot: 30-120 parts; (5) bullacta: 30-60 parts; (6) sub complanatus: 6-9 parts; (7) dodder: 6-15 parts; more than the one kind can be selected from above 7 kinds of the raw materials for the liver nourishing and the eyesight improving and added into the actual formulation group;
   the herbal confectionery candy for eyesight improving also comprises following component parts by a mass of a sixth type of the raw materials used for dispelling wind and the eyesight improving:
      (1) aeschynomene: 15-30 parts; (2) albizia flower: 3-9 parts; (3) sallucidum: 0.9-1.5 parts; (4) tribulus terrestris: 6-9 parts; (5) sub shepherd's purse: 10-30 parts; (6) hydrangea wind: 15-30 parts; (7) safflower vegetable: 15-30 parts; (8) astragalus sinicus seed: 6-9 parts; (9) herba setaria viridis: 6-12 parts; more than the one kind can be selected from above 9 kinds of the raw materials for the liver nourishing and the eyesight improving and added into the actual formulation group;
      in above 54 kinds of the raw materials from the second type of the raw materials to the sixth type of the raw materials, the ginkgo leaves and the wood butterfly must be used together for stronger effect and a neither should be omitted; in and in the above 54 raw materials from the second type of the raw materials to the sixth type of the raw materials, more than 7 kinds out of the 54 kinds of the raw materials from the second type of the raw materials to the sixth type of the raw materials are selected to added into the actual formulation group, the raw materials of the actual formulation group need to be respectively cut into filaments or pulverized into granules to improve decoction extraction efficiency; decoction liquid plus sugar is concentrated into paste by decompressing inner ebullition method; during a cooling and solidification process, the herbal materials are pressed to form a symmetrical two-half oval capsule shell container, and a weight of two half capsule is between 0.8 to 4g.

The creative features of this invention are as follows: Based on the extensive collection of American, European and Chinese ophthalmic medicinal materials world widely, select 66 ophthalmic outstanding raw materials and classify them into six major categories, with reference to Capsule dosage form technology from Western medicine and candy preparation method from China, creatively develop the core technical having advantages of both above two unique production processes, combine the best human ophthalmic medicinal materials of both Chinese and Western medicine and achieve the aim of this invention.

Annotations for special text symbol terms appearing in this instruction:
the amount of each raw material used, and the range in which the amount of used can vary, are expressed in parts by weight, referred to as "parts".

### DETAILED DESCRIPTION OF THE EMBODIMENTS

The present invention will be further described with specific embodiments:
Embodiment 1. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, flos eriocauli 9-12 parts, equisetum 3-10 parts, buddleia 6-15 parts, emblica cassia 6-15 parts, rabbit liver 30-60 parts, nux prinsepiae 3-10 parts, aeschynomene 15-30 parts, Albizia flower 3-9 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group A, sealed the two halves of capsule to obtain soft capsule products ready for packaging and discharging.
Embodiment 2. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, flos eriocauli 9-12 parts, goat liver 30-60 parts, buddleia 6-15 parts, abalone shell 10-30 parts, rabbit liver 30-60 parts, carrot 30-120 parts, aeschynomene 15-30 parts, sallucidum 0.9-1.5 parts, these 12 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method, after the cooling and solidification process, pressed into a symmetrical two-half oval capsule shell, which will be filled with candy core tablet made of human lens and retinal photoreceptor nutrients in group A, sealed the two halves of capsule to obtain soft capsule products ready for packaging and discharging.
Embodiment 3. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, flos eriocauli 9-12 parts, semen thlaspi 5-15 parts, buddleia 6-15 parts, yemingsha 3-10 parts, rabbit liver 30-60 parts, bullacta 30-60 parts, aeschynomene 15-30 parts, tribulus terrestris 6-9 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group A, sealed the two halves of capsule to obtain soft capsule products ready for packaging and discharging.
Embodiment 4. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, flos eriocauli 9-12 parts, ghost eye 6 parts, buddleia 6-15 parts, sophora fruit 5-15 parts, rabbit liver 30-60 parts, sub complanatus 6-9 parts, aeschynomene 15-30 parts, sub shepherd's purse 10-30 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group A, sealed the two halves of capsule to obtain soft capsule products ready for packaging and discharging.
Embodiment 5. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, flos eriocauli 9-12 parts, equisetum 3-10 parts, buddleia 6-15 parts, emblica cassia6-15 parts, rabbit liver 30-60 parts, nux prinsepiae 3-10 parts, aeschynomene 15-30 parts, Albizia flower 3-9 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 6. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, flos eriocauli 9-12 parts, semen thlaspi 5-15 parts, buddleia 6-15 parts, abalone shell 10-30 parts, rabbit liver 30-60 parts, carrot 30-120 parts, aeschynomene 15-30 parts, sallucidum 0.9-1.5 parts, these 12 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 7. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, flos eriocauli 9-12 parts, ghost eye 6 parts, buddleia 6-15 parts, yemingsha 3-10 parts, rabbit liver 30-60 parts, bullacta 30-60 parts, aeschynomene 15-30 parts, tribulus terrestris 6-9 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 8. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, flos eriocauli 9-12 parts, chrysanthemum 10-15 parts, buddleia 6-15 parts, sophora fruit 5-15 parts, rabbit liver 30-60 parts, sub complanatus 6-9 parts, aeschynomene 15-30 parts, sub shepherd's purse 10-30 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 9. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, flos eriocauli 9-12 parts, basil 3-5 parts, buddleia 6-15 parts, glossy privet leaves 10-15 parts, rabbit liver 30-60 parts, dodder 6-15 parts, aeschynomene 15-30 parts, hydrangea wind 15-30 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 10. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, flos eriocauli 9-12 parts, fructus rubi careani 9-15 parts, buddleia 6-15 parts, herb blumeae megacephalae 10-15 parts, rabbit liver 30-60 parts, carrot 30-120 parts, aeschynomene 15-30 parts, safflower vegetable 15-30 parts, these 12 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 11. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, flos eriocauli 9-12 parts, equisetum 3-10 parts, buddleia 6-15 parts, emblica cassia6-15 parts, rabbit liver 30-60 parts, nux prinsepiae 3-10 parts, aeschynomene 15-30 parts, Albizia flower 3-9 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 12. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, flos eriocauli 9-12 parts, semen thlaspi 5-15 parts, buddleia 6-15 parts, abalone shell 10-30 parts, rabbit liver 30-60 parts, carrot 30-120 parts, aeschynomene 15-30 parts, sallucidum 0.9-1.5 parts, these 12 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 13. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, flos eriocauli 9-12 parts, ghost eye 6 parts, buddleia 6-15 parts, yemingsha 3-10 parts, rabbit liver 30-60 parts, bullacta 30-60 parts, aeschynomene 15-30 parts, tribulus terrestris 6-9 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 14. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, flos eriocauli 9-12 parts, chrysanthemum 10-15 parts, buddleia 6-15 parts, sophora fruit 5-15 parts, rabbit liver 30-60 parts, sub complanatus 6-9 parts, aeschynomene 15-30 parts, sub shepherd's purse 10-30 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 15. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, flos eriocauli 9-12 parts, basil 3-5 parts, buddleia 6-15 parts, glossy privet leaves 10-15 parts, rabbit liver 30-60 parts, dodder 6-15 parts, aeschynomene 15-30 parts, hydrangea wind 15-30 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 16. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, flos eriocauli 9-12 parts, fructus rubi careani 9-15 parts, buddleia 6-15 parts, herb blumeae megacephalae 10-15 parts, rabbit liver 30-60 parts, carrot 30-120 parts, aeschynomene 15-30 parts, safflower vegetable 15-30 parts, these 12 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 17. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, flos eriocauli 9-12 parts, equisetum 3-10 parts, buddleia 6-15 parts, emblica cassia6-15 parts, rabbit liver 30-60 parts, nux prinsepiae 3-10 parts, aeschynomene 15-30 parts, Albizia flower 3-9 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 18. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, flos eriocauli 9-12 parts, semen thlaspi 5-15 parts, buddleia 6-15 parts, abalone shell 10-30 parts, rabbit liver 30-60 parts, carrot 30-120 parts, aeschynomene 15-30 parts, sallucidum 0.9-1.5 parts, these 12 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 19. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, flos eriocauli 9-12 parts, ghost eye 6 parts, buddleia 6-15 parts, yemingsha 3-10 parts, rabbit liver 30-60 parts, bullacta 30-60 parts, aeschynomene 15-30 parts, tribulus terrestris 6-9 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 20. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, flos eriocauli 9-12 parts, chrysanthemum 10-15 parts, buddleia 6-15 parts, sophora fruit 5-15 parts, rabbit liver 30-60 parts, sub complanatus 6-9 parts, aeschynomene 15-30 parts, sub shepherd's purse 10-30 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 21. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, flos eriocauli 9-12 parts, basil 3-5 parts, buddleia 6-15 parts, glossy privet leaves 10-15 parts, rabbit liver 30-60 parts, dodder 6-15 parts, aeschynomene 15-30 parts, hydrangea wind 15-30 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 22. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, flos eriocauli 9-12 parts, fructus rubi careani 9-15 parts, buddleia 6-15 parts, herb blumeae megacephalae 10-15 parts, rabbit liver 30-60 parts, carrot 30-120 parts, aeschynomene 15-30 parts, safflower vegetable 15-30 parts, these 12 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 23. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, flos eriocauli 9-12 parts, equisetum 3-10 parts, buddleia 6-15 parts, emblica cassia6-15 parts, rabbit liver 30-60 parts, nux prinsepiae 3-10 parts, aeschynomene 15-30 parts, Albizia flower 3-9 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 24. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, flos eriocauli 9-12 parts, semen thlaspi 5-15 parts, buddleia 6-15 parts, abalone shell 10-30 parts, rabbit liver 30-60 parts, carrot 30-120 parts, aeschynomene 15-30 parts, sallucidum 0.9-1.5 parts, these 12 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 25. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, flos eriocauli 9-12 parts, ghost eye 6 parts, buddleia 6-15 parts, yemingsha 3-10 parts, rabbit liver 30-60 parts, bullacta 30-60 parts, aeschynomene 15-30 parts, tribulus terrestris 6-9 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 26. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, flos eriocauli 9-12 parts, chrysanthemum 10-15 parts, buddleia 6-15 parts, sophora fruit 5-15 parts, rabbit liver 30-60 parts, sub complanatus 6-9 parts, aeschynomene 15-30 parts, sub shepherd's purse 10-30 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 27. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, flos eriocauli 9-12 parts, basil 3-5 parts, buddleia 6-15 parts, glossy privet leaves 10-15 parts, rabbit liver 30-60 parts, dodder 6-15 parts, aeschynomene 15-30 parts, hydrangea wind 15-30 parts, these 12 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 28. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, flos eriocauli 9-12 parts, fructus rubi careani 9-15 parts, buddleia 6-15 parts, herb blumeae megacephalae 10-15 parts, rabbit liver 30-60 parts, carrot 30-120 parts, aeschynomene 15-30 parts, safflower vegetable 15-30 parts, these 12 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method, after the cooling and solidification process, pressed into a symmetrical two-half oval capsule shell, which will be filled with candy core tablet made of human lens and retinal photoreceptor nutrients in group B, sealed the two halves of capsule to obtain soft capsule products ready for packaging and discharging.
Embodiment 29. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, goat liver 30-60 parts, dream flowers 3-15 parts, buddleia 6-15 parts, emblica cassia6-15 parts, nux prinsepiae 3-10 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 30. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, goat liver 30-60 parts, flos eriocauli 9-12 parts, buddleia 6-15 parts, abalone shell 10-30 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 31. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, goat liver 30-60 parts, equisetum 3-10 parts, buddleia 6-15 parts, yemingsha 3-10 parts, carrot 30-120 parts, sallucidum 0.9-1.5 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 32. A herbal confectionery candy for eyesight improvingdescribed previously in summary, wherein the parts prepared from the following raw Chinese medicine materials in weight: ginkgo leaves 3-9 parts, wood butterfly 6-9 parts, ligustrum fruit 6-15 parts, goat liver 30-60 parts, semen thlaspi 5-15 parts, buddleia 6-15 parts, sophora fruit 5-15 parts, carrot 30-120 parts, tribulus terrestris 6-9 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 33. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, goat liver 30-60 parts, ghost eye 6 parts, buddleia 6-15 parts, glossy privet leaves 10-15 parts, carrot 30-120 parts, sub shepherd's purse 10-30 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 34. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, goat liver 30-60 parts, chrysanthemum 10-15 parts, buddleia 6-15 parts, herb blumeae megacephalae 10-15 parts, bullacta 30-60 parts, hydrangea wind 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 35. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, goat liver 30-60 parts, basil 3-5 parts, buddleia 6-15 parts, ash bark 6-12 parts, sub complanatus 6-9 parts, safflower vegetable 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 36. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, goat liver 30-60 parts, fructus rubi careani 9-15 parts, buddleia 6-15 parts, medlar 5-15 parts, dodder 6-15 parts, astragalus sinicus seed 6-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 37. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, flos eriocauli 9-12 parts, buddleia 6-15 parts, emblica cassia6-15 parts, rabbit liver 30-60 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 38. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, equisetum 3-10 parts, buddleia 6-15 parts, abalone shell 10-30 parts, rabbit liver 30-60 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 39. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, semen thlaspi 5-15 parts, buddleia 6-15 parts, yemingsha 3-10 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 40. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, ghost eye 6 parts, buddleia 6-15 parts, sophora fruit 5-15 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 41. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, goat liver 30-60 parts, dream flowers 3-15 parts, buddleia 6-15 parts, emblica cassia6-15 parts, nux prinsepiae 3-10 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 42. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, goat liver 30-60 parts, flos eriocauli 9-12 parts, buddleia 6-15 parts, abalone shell 10-30 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 43. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, goat liver 30-60 parts, equisetum 3-10 parts, buddleia 6-15 parts, yemingsha 3-10 parts, carrot 30-120 parts, sallucidum 0.9-1.5 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 44. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, goat liver 30-60 parts, semen thlaspi 5-15 parts, buddleia 6-15 parts, sophora fruit 5-15 parts, carrot 30-120 parts, tribulus terrestris 6-9 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 45. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, goat liver 30-60 parts, ghost eye 6 parts, buddleia 6-15 parts, glossy privet leaves 10-15 parts, carrot 30-120 parts, sub shepherd's purse 10-30 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 46. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, goat liver 30-60 parts, chrysanthemum 10-15 parts, buddleia 6-15 parts, herb blumeae megacephalae 10-15 parts, bullacta 30-60 parts, hydrangea wind 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 47. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, goat liver 30-60 parts, basil 3-5 parts, buddleia 6-15 parts, ash bark 6-12 parts, sub complanatus 6-9 parts, safflower vegetable 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 48. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, goat liver 30-60 parts, fructus rubi careani 9-15 parts, buddleia 6-15 parts, medlar 5-15 parts, dodder 6-15 parts, astragalus sinicus seed 6-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 49. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, flos eriocauli 9-12 parts, buddleia 6-15 parts, emblica cassia6-15 parts, rabbit liver 30-60 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 50. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, equisetum 3-10 parts, buddleia 6-15 parts, abalone shell 10-30 parts, rabbit liver 30-60 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 51. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, semen thlaspi 5-15 parts, buddleia 6-15 parts, yemingsha 3-10 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 52. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, dream flowers 3-15 parts, ghost eye 6 parts, buddleia 6-15 parts, sophora fruit 5-15 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 53. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, goat liver 30-60 parts, dream flowers 3-15 parts, buddleia 6-15 parts, emblica cassia6-15 parts, nux prinsepiae 3-10 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 54. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, goat liver 30-60 parts, flos eriocauli 9-12 parts, buddleia 6-15 parts, abalone shell 10-30 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 55. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, goat liver 30-60 parts, equisetum 3-10 parts, buddleia 6-15 parts, yemingsha 3-10 parts, carrot 30-120 parts, sallucidum 0.9-1.5 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 56. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, goat liver 30-60 parts, semen thlaspi 5-15 parts, buddleia 6-15 parts, sophora fruit 5-15 parts, carrot 30-120 parts, tribulus terrestris 6-9 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 57. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, goat liver 30-60 parts, ghost eye 6 parts, buddleia 6-15 parts, glossy privet leaves 10-15 parts, carrot 30-120 parts, sub shepherd's purse 10-30 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 58. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, goat liver 30-60 parts, chrysanthemum 10-15 parts, buddleia 6-15 parts, herb blumeae megacephalae 10-15 parts, bullacta 30-60 parts, hydrangea wind 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 59. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, goat liver 30-60 parts, basil 3-5 parts, buddleia 6-15 parts, ash bark 6-12 parts, sub complanatus 6-9 parts, safflower vegetable 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 60. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, goat liver 30-60 parts, fructus rubi careani 9-15 parts, buddleia 6-15 parts, medlar 5-15 parts, dodder 6-15 parts, astragalus sinicus seed 6-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 61. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, flos eriocauli 9-12 parts, buddleia 6-15 parts, emblica cassia6-15 parts, rabbit liver 30-60 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 62. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, equisetum 3-10 parts, buddleia 6-15 parts, abalone shell 10-30 parts, rabbit liver 30-60 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 63. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, semen thlaspi 5-15 parts, buddleia 6-15 parts, yemingsha 3-10 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 64. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, dream flowers 3-15 parts, ghost eye 6 parts, buddleia 6-15 parts, sophora fruit 5-15 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 65. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, goat liver 30-60 parts, dream flowers 3-15 parts, buddleia 6-15 parts, emblicacassia6-15 parts, nux prinsepiae 3-10parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 66. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, goat liver 30-60 parts, flos eriocauli 9-12 parts, buddleia 6-15 parts, abalone shell 10-30 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 67. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, goat liver 30-60 parts, equisetum 3-10 parts, buddleia 6-15 parts, yemingsha 3-10 parts, carrot 30-120 parts, sallucidum 0.9-1.5 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 68. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, goat liver 30-60 parts, semen thlaspi 5-15 parts, buddleia 6-15 parts, sophora fruit 5-15 parts, carrot 30-120 parts, tribulus terrestris 6-9 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 69. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, goat liver 30-60 parts, ghost eye 6 parts, buddleia 6-15 parts, glossy privet leaves 10-15 parts, carrot 30-120 parts, sub shepherd's purse 10-30 parts, these 9 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 70. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, goat liver 30-60 parts, chrysanthemum 10-15 parts, buddleia 6-15 parts, herb blumeae megacephalae 10-15 parts, bullacta 30-60 parts, hydrangea wind 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 71. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, goat liver 30-60 parts, basil 3-5 parts, buddleia 6-15 parts, ash bark 6-12 parts, sub complanatus 6-9 parts, safflower vegetable 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 72. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, goat liver 30-60 parts, fructus rubi careani 9-15 parts, buddleia 6-15 parts, medlar 5-15 parts, dodder 6-15 parts, astragalus sinicus seed 6-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 73. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, flos eriocauli 9-12 parts, buddleia 6-15 parts, emblica cassia6-15 parts, rabbit liver 30-60 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 74. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, equisetum 3-10 parts, buddleia 6-15 parts, abalone shell 10-30 parts, rabbit liver 30-60 parts, aeschynomene 15-30 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 75. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, semen thlaspi 5-15 parts, buddleia 6-15 parts, yemingsha 3-10 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 76. A herbal confectionery candy for eyesight improving described previously in summary, wherein the parts prepared from the following raw Chinese medicine materials in weight: ginkgo leaves 3-9 parts, wood butterfly 6-9 parts, dendrobium 6-15 parts, dream flowers 3-15 parts, ghost eye 6 parts, buddleia 6-15 parts, sophora fruit 5-15 parts, nux prinsepiae 3-10 parts, Albizia flower 3-9 parts, these 9 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group C, sealed the two halves of capsule to obtain soft capsule products ready for packaging and discharging.
Embodiment 77. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, buddleia 6-15 parts, carrot 30-120 parts, aeschynomene 15-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 78. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, flos eriocauli 9-12 parts, emblica cassia6-15 parts, carrot 30-120 parts, Albizia flower 3-9 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 79. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, equisetum 3-10 parts, abalone shell 10-30 parts, carrot 30-120 parts, sallucidum 0.9-1.5 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 80. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, semen thlaspi 5-15 parts, yemingsha 3-10 parts, carrot 30-120 parts, tribulus terrestris 6-9 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 81. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, ghost eye 6 parts, sophora fruit 5-15 parts, carrot 30-120 parts, sub shepherd's purse 10-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 82. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, chrysanthemum 10-15 parts, glossy privet leaves 10-15 parts, carrot 30-120 parts, hydrangea wind 15-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 83. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, basil 3-5 parts, herb blumeae megacephalae 10-15 parts, carrot 30-120 parts, safflower vegetable 15-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 84. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, fructus rubi careani 9-15 parts, ash bark 6-12 parts, carrot 30-120 parts, astragalus sinicus seed 6-9 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 85. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, herba sceptridii 6-12 parts, fresh medlar leaf 60-240 parts, carrot 30-120 parts, herba setaria viridis 6-12 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 86. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, raspberry root 5-10 parts, medlar 5-15 parts, carrot 30-120 parts, aeschynomene 15-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 87. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, semen allii fistulosi 6-12 parts, prunella 6-15 parts, carrot 30-120 parts, aeschynomene 15-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 88. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, senecio 15-30 parts, abalone shell 10-30 parts, carrot 30-120 parts, aeschynomene 15-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 89. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, plantago 5-15 parts, abalone shell10-3,carrot 30-120 parts, sallucidum 0.9-1.5 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 90. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, equisetum 3-10 parts, sophora fruit 5-15 parts, carrot 30-120 parts, aeschynomene 15-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 91. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, ghost eye 6 parts, glossy privet leaves 10-15 parts, carrot 30-120 parts, sub shepherd's purse 10-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 92. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, buckwheat leaves 5-10 parts, herb blumeae megacephalae 10-15 parts, carrot 30-120 parts, hydrangea wind 15-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 93. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, black bean 5-10 parts, ash bark 6-12 parts, carrot 30-120 parts, safflower vegetable 15-30 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 94. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, raspberry root 15-30 parts, fresh medlar leaf 60-240 parts, carrot 30-120 parts, sallucidum 0.9-1.5 parts, these 7 raw materials were cut into filaments or pulverized into granules to improve the decoction extraction efficiency, squeezed washed carrots into juice and standby, decocted the chopped carrot dregs, combined the extract with the juice, concentrated the total herbal decoction liquid with sugar added into paste with Decompressing Inner Ebullition method , after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 95. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, buddleia 6-15 parts, nux prinsepiae 3-10 parts, aeschynomene 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 96. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, flos eriocauli 9-12 parts, emblica cassia6-15 parts, nux prinsepiae 3-10 parts, Albizia flower 3-9 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 97. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, equisetum 3-10 parts, abalone shell 10-30 parts, nux prinsepiae 3-10 parts, sallucidum 0.9-1.5 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 98. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, semen thlaspi 5-15 parts, yemingsha 3-10 parts, nux prinsepiae 3-10 parts, tribulus terrestris 6-9 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 99. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, ghost eye 6 parts, sophora fruit 5-15 parts, nux prinsepiae 3-10 parts, sub shepherd's purse 10-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 100. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, chrysanthemum 10-15 parts, glossy privet leaves 10-15 parts, nux prinsepiae 3-10 parts, hydrangea wind 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 101. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, basil 3-5 parts, herb blumeae megacephalae 10-15 parts, nux prinsepiae 3-10 parts, safflower vegetable 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 102. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, fructus rubi careani 9-15 parts, ash bark 6-12 parts, nux prinsepiae 3-10 parts, astragalus sinicus seed 6-9 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 103. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, herba sceptridii 6-12 parts, fresh medlar leaves 60-240 parts, nux prinsepiae 3-10 parts, herba setaria viridis 6-12 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 104. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, raspberry 5-10 parts, medlar 5-15 parts, nux prinsepiae 3-10 parts, aeschynomene 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 105. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, semen allii fistulosi 6-12 parts, prunella 6-15 parts, nux prinsepiae 3-10 parts, aeschynomene 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 106. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, senecio 15-30 parts, abalone shell 10-30 parts, nux prinsepiae 3-10 parts, aeschynomene 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 107. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, plantago 5-15 parts, abalone shell10-3,nux prinsepiae 3-10 parts, sallucidum 0.9-1.5 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 108. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, equisetum 3-10 parts, sophora fruit 5-15 parts, nux prinsepiae 3-10 parts, aeschynomene 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 109. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, ghost eye 6 parts, glossy privet leaves 10-15 parts, nux prinsepiae 3-10 parts, sub shepherd's purse 10-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 110. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, buckwheat leaves 5-10 parts, herb blumeae megacephalae 10-15 parts, nux prinsepiae 3-10 parts, hydrangea wind 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 111. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, black bean 5-10 parts, ash bark 6-12 parts, nux prinsepiae 3-10 parts, safflower vegetable 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 112. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, raspberry root 15-30 parts, fresh medlar leaves 60-240 parts, nux prinsepiae 3-10 parts, sallucidum 0.9-1.5 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 113. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, dream flowers 3-15 parts, buddleia 6-15 parts, pork liver 60-150 parts, aeschynomene 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 114. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, flos eriocauli 9-12 parts, emblica cassia6-15 parts, pork liver 60-150 parts, Albizia flower 3-9 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 115. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, equisetum 3-10 parts, abalone shell 10-30 parts, pork liver 60-150 parts, sallucidum 0.9-1.5 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 116. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, semen thlaspi 5-15 parts, yemingsha 3-10 parts, pork liver 60-150 parts, tribulus terrestris 6-9 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 117. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, ghost eye 6 parts, sophora fruit 5-15 parts, pork liver 60-150 parts, sub shepherd's purse 10-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 118. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, chrysanthemum 10-15 parts, glossy privet leaves 10-15 parts, pork liver 60-150 parts, hydrangea wind 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 119. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, basil 3-5 parts, herb blumeae megacephalae 10-15 parts, pork liver 60-150 parts, safflower vegetable 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 120. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, fructus rubi careani 9-15 parts, ash bark 6-12 parts, pork liver 60-150 parts, astragalus sinicus seed 6-9 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 121. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, herba sceptridii 6-12 parts, fresh medlar leaves 60-240 parts, pork liver 60-150 parts, herba setaria viridis 6-12 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 122. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, raspberry 5-10 parts, medlar 5-15 parts, pork liver 60-150 parts, aeschynomene 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 123. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, semen allii fistulosi 6-12 parts, prunella 6-15 parts, pork liver 60-150 parts, aeschynomene 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 124. A herbal confectionery candy for eyesight improving described previously in summary, wherein the parts prepared from the following raw Chinese medicine materials in weight: ginkgo leaves 3-9 parts, wood butterfly 6-9 parts, ligustrum fruit 6-15 parts, senecio 15-30 parts, paper mulberry fruit6-10 parts, pork liver 60-150 parts, sallucidum 0.9-1.5 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 125. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, plantago 5-15 parts, abalone shell10-3,pork liver 60-150 parts, sallucidum 0.9-1.5 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 126. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, phyllanthus 15-30 parts, sophora fruit 5-15 parts, pork liver 60-150 parts, tribulus terrestris 6-9 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 127. A herbal confectionery candy for eyesight improving described previously in summary, wherein the parts prepared from the following raw Chinese medicine materials in weight: ginkgo leaves 3-9 parts, wood butterfly 6-9 parts, feather cockscomb seed 3-15 parts, buckwheat leaves 5-10 parts, glossy privet leaves 10-15 parts, pork liver 60-150 parts, sub shepherd's purse 10-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 128. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, buckwheat leaves 5-10 parts, herb blumeae megacephalae 10-15 parts, pork liver 60-150 parts, hydrangea wind 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 129. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, black bean 5-10 parts, ash bark 6-12 parts, pork liver 60-150 parts, safflower vegetable 15-30 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.
Embodiment 130. A herbal confectionery candy for eyesight improving described previously in summary, wherein the parts prepared from the following raw Chinese medicine materials in weight: ginkgo leaves 3-9 parts, wood butterfly 6-9 parts, nacres 10-30 parts, raspberry root 15-30 parts, fresh medlar leaves 60-240 parts, pork liver 60-150 parts, sallucidum 0.9-1.5 parts, these 7 raw materials were cut into filaments or pulverized into granules to improve the decoction extraction efficiency, added sugar into the decoction liquid and concentrated the mixture into paste with Decompressing Inner Ebullition method, after the cooling and solidification process, pressed into a symmetrical two-half oval capsule shell, which will be filled with candy core tablet made of human lens and retinal photoreceptor nutrients in group D, sealed the two halves of capsule to obtain soft capsule products ready for packaging and discharging.

## Claims

1. An herbal confectionery candy for eyesight improving, wherein: the herbal confectionery candy comprises a first type of raw materials composed of human eye lens and retinal photoreceptor cells nutrients, comprising:
(1) rhodopsin nucleotides: 0.0002-0.001 parts; (2) 11-cis-retinal: 0.0002-0.001 parts; (3) lutein ester: 0.001-0.006 parts; (4) zeaxanthin: 0.001-0.005 parts; (5) accinium myrtillus anthocyanin: 0.03-0.15 parts; (6) blueberry anthocyanin: 0.04-0.22 parts; (7) β-carotene: 0.001-0.006 parts; (8) vitamin B2: 0.0004-0.002 parts; (9) docosahexaenoic acid: 0.04-0.22 parts; (10) vitamin C: 0.06-0.3 parts; (11) haematococcus pluvialis: 0.06-0.3 parts; (12) blueberry powder: 0.2-1 parts;
more than 5 kinds can be selected from above 12 kinds of the human eye lens and retinal photoreceptor cells nutrients and added into an actual formulation group, and a total weight of selected raw materials should be between 0.3g and 1.8g;
the actual formulation group comprises following groups: 11 kinds of the human eye lens and retinal photoreceptor cells nutrients are selected from (1) to (11) as group A, 10 kinds of the human eye lens and retinal photoreceptor cells nutrients are selected from (2) to (11) as group B, 8 kinds of the human eye lens and retinal photoreceptor cells nutrients:(3), (4), (6), (7), (8), (9), (10), (11) are selected as group C, 5 kinds of the human eye lens and retinal photoreceptor cells nutrients: (3), (7), (10), (11), (12) are selected as group D; the selected raw materials in all the groups must can be pressed into a flat oval shape candy core tablet with a designed size for use, and the flat oval shape candy core tablet should be able to be filled into a flat shell-shaped candy capsule to complete a confectionery product;
the herbal confectionery candy for eyesight improving also comprises following component parts by a mass of a second type of the raw materials used against eye damage:
(1) ginkgo leaves: 3-9 parts; (2) wood butterfly: 6-9 parts; (3) ligustrum fruit: 6-15 parts; (4) feather cockscomb seed: 3-15 parts; (5) mother of pearl: 10-30 parts; (6) dendrobium: 6-15 parts; more than 3 kinds can be selected from above 6 kinds of the raw materials used against eye damage and added into the actual formulation group, wherein, the (1) ginkgo leaves and the (2) wood butterfly must be selected as the raw materials of the actual formulation group;
the herbal confectionery candy for eyesight improving also comprises following component parts by a mass of a third type of the raw materials used for the eyesight improving:
(1) dream flower: 3-15 parts; (2) flos eriocauli: 9-12 parts; (3) equisetum: 3-10 parts; (4) goat liver: 30-60 parts; (5) semen thlaspi: 5-15 parts; (6) ghost eye: 6 parts; (7) chrysanthemum: 10-15 parts; (8) basil: 3-5 parts; (9) fructus rubi careani: 9-15 parts; (10) herba sceptridii: 6-12 parts; (11) raspberry: 5-10 parts; (12) semen allii fistulosi: 6-12 parts; (13) senecio: 15-30 parts; (14) plantago: 5-15 parts; (15) black soya bean flower: 3-9 parts; (16) vitis romanetii roman: 15-30 parts; (17) phyllanthus: 15-30 parts; (18) buckwheat leaves: 5-10 parts; (19) black bean: 5-10 parts; (20) raspberry root: 15-30 parts; more than an one kind can be selected from above 20 kinds of the raw materials for the eyesight improving and added into the actual formulation group;
the herbal confectionery candy for eyesight improving also comprises following component parts by a mass of a fourth type of the raw materials used for liver clearing and the eyesight improving:
(1) buddleia: 6-15 parts; (2) cassia: 6-30 parts; (3) abalone shell: 10-30 parts; (4) yemingsha: 3-10 parts; (5) sophora fruit: 5-15 parts; (6) glossy privet leaves: 10-15 parts; (7) herba blumeae megacephalae: 10-15 parts; (8) qinpi: 6-12 parts; (9) medlar leaf: fresh leaves 60-240 parts; (10) medlar: 5-15 parts; (11) prunella: 6-30 parts; (12) paper mulberry fruit: 6-10 parts; more than the one kind can be selected from above 12 kinds of the raw materials for the liver clearing and the eyesight improving and added into the actual formulation group;
the herbal confectionery candy for eyesight improving also comprises following component parts by a mass of a fifth type of the raw materials used for liver nourishing and the eyesight improving:
(1) rabbit liver: 30-60 parts; (2) nux prinsepiae: 3-10 parts; (3) pork liver: 60-150 parts; (4) carrot: 30-120 parts; (5) bullacta: 30-60 parts; (6) sub complanatus: 6-9 parts; (7) dodder: 6-15 parts; more than the one kind can be selected from above 7 kinds of the raw materials for the liver nourishing and the eyesight improving and added into the actual formulation group;
the herbal confectionery candy for eyesight improving also comprises following component parts by a mass of a sixth type of the raw materials used for dispelling wind and the eyesight improving:
(1) aeschynomene: 15-30 parts; (2) albizia flower: 3-9 parts; (3) sallucidum: 0.9-1.5 parts; (4) tribulus terrestris: 6-9 parts; (5) sub shepherd's purse: 10-30 parts; (6) hydrangea wind: 15-30 parts; (7) safflower vegetable: 15-30 parts; (8) astragalus sinicus seed: 6-9 parts; (9) herba setaria viridis: 6-12 parts; more than the one kind can be selected from above 9 kinds of the raw materials for the liver nourishing and the eyesight improving and added into the actual formulation group;
in above 54 kinds of the raw materials from the second type of the raw materials to the sixth type of the raw materials, the ginkgo leaves and the wood butterfly must be used together for stronger effect and a neither should be omitted; in and in the above 54 raw materials from the second type of the raw materials to the sixth type of the raw materials, more than 7 kinds out of the 54 kinds of the raw materials from the second type of the raw materials to the sixth type of the raw materials are selected to added into the actual formulation group, the raw materials of the actual formulation group need to be respectively cut into filaments or pulverized into granules to improve decoction extraction efficiency; decoction liquid plus sugar is concentrated into paste by decompressing inner ebullition method; during a cooling and solidification process, the herbal materials are pressed to form a symmetrical two-half oval capsule shell container, and a weight of two half capsule is between 0.8 to 4g.

2. The herbal confectionery candy for eyesight improving according to claim 1, wherein: selected Chinese herbal raw materials are: the ginkgo leaves: 3-9 parts, the wood butterfly: 6-9 parts, the ligustrum fruit: 6-15 parts, the dream flowers: 3-15 parts, the flos eriocauli: 9-12 parts, the equisetum: 3-10 parts, the buddleia: 6-15 parts, the cassia: 6-15 parts, the rabbit liver: 30-60 parts, the nux prinsepiae: 3-10 parts, the aeschynomene: 15-30 parts, and the albizia flower: 3-9 parts, 12 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with a candy core tablet made of a human lens and retinal photoreceptor nutrients in a group A, and two halves oval capsule are sealed by the paste to obtain soft capsule products ready for packaging and shipping.

3. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the dream flowers: 3-15 parts, the flos eriocauli: 9-12 parts, the equisetum: 3-10 parts, the buddleia: 6-15 parts, the cassia: 6-15 parts, the rabbit liver: 30-60 parts, the nux prinsepiae: 3-10 parts, the aeschynomene: 15-30 parts, and the albizia flower: 3-9 parts, the 12 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with a candy core tablet made of a human lens and retinal photoreceptor nutrients in a group B, and the two halves oval capsule are sealed by the paste to obtain the soft capsule products ready for packaging and shipping.

4. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the dream flowers: 3-15 parts, the flos eriocauli: 9-12 parts, the semen thlaspi: 5-15 parts, the buddleia: 6-15 parts, the abalone shell: 10-30 parts, the rabbit liver: 30-60 parts, the carrot: 30-120 parts, the aeschynomene: 15-30 parts, and the sallucidum: 0.9-1.5 parts, the 12 Chinese herbal raw materials are cut into the filaments or pulverized into the granules to improve the decoction extraction efficiency, the carrot is washed and squeezed into juice for using, carrot dregs are cut and extracted by the decoction, and carrot decoction liquid is mixed with the juice; all the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group B, and the two halves oval capsule are sealed by the paste to obtain the soft capsule products ready for packaging and shipping.

5. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the dream flowers: 3-15 parts, the flos eriocauli: 9-12 parts, the ghost mesh: 6 parts, the buddleia: 6-15 parts, the yemingsha: 3-10 parts, the rabbit liver: 30-60 parts, the bullacta: 30-60 parts, the aeschynomene: 15-30 parts, and the tribulus terrestris: 6-9 parts, the 12 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group B, and the two halves oval capsule are sealed by the paste to obtain the soft capsule products ready for packaging and shipping.

6. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the dream flowers: 3-15 parts, the flos eriocauli: 9-12 parts, the chrysanthemum: 10-15 parts, the buddleia: 6-15 parts, the sophora fruit: 5-15 parts, the rabbit liver: 30-60 parts, the sub complanatus: 6-9 parts, the aeschynomene: 15-30 parts, and the sub shepherd's purse: 10-30 parts, the 12 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group B, and the two halves oval capsule are sealed by the paste to obtain the soft capsule products ready for packaging and shipping.

7. The herbal confectionery candy for eyesight improving according to claim 1, wherein: the selected Chinese herbal raw materials are: the ginkgo leaves: 3-9 parts, the wood butterfly: 6-9 parts, the feather cockscomb seed: 3-15 parts, the dream flowers: 3-15 parts, the flos eriocauli: 9-12 parts, the equisetum: 3-10 parts, the buddleia: 6-15 parts, the cassia: 6-15 parts, the rabbit liver: 30-60 parts, the nux prinsepiae: 3-10 parts, the aeschynomene: 15-30 parts, and the albizia flower: 3-9 parts, the 12 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group B, and the two halves oval capsule are sealed by the paste to obtain the soft capsule products ready for packaging and shipping.

8. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the goat liver: 30-60 parts and the dream flowers: 3-15 parts, the buddleia: 6-15 parts, the cassia: 6-15 parts, the nux prinsepiae: 3-10 parts, and the aeschynomene: 15-30 parts, 9 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with a candy core tablet made of a human lens and retinal photoreceptor nutrients in a group C, and the two halves oval capsule are sealed by the paste to obtain the soft capsule products ready for packaging and shipping.

9. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the goat liver: 30-60 parts, the flos eriocauli: 9-12 parts, the buddleia: 6-15 parts, the abalone shell: 10-30 parts, the nux prinsepiae: 3-10 parts, and the albizia flower: 3-9 parts, the 9 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group C, and the two halves oval capsule are sealed by the paste to obtain the soft capsule products ready for packaging and shipping.

10. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the dream flowers: 3-15 parts, and the flos eriocauli: 9-12 parts, the buddleia: 6-15 parts, the cassia: 6-15 parts, the rabbit liver: 30-60 parts, and the aeschynomene: 15-30 parts, the 9 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group C, and the two halves oval capsule are sealed by the paste to obtain the soft capsule products ready for packaging and shipping.

11. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the dream flowers: 3-15 parts, and the equisetum: 3-10 parts, the buddleia: 6-15 parts, the abalone shell: 10-30 parts, the rabbit liver: 30-60 parts, and the aeschynomene: 15-30 parts, the 9 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group C, and the two halves oval capsule are sealed by the paste to obtain the soft capsule products ready for packaging and shipping.

12. The herbal confectionery candy for eyesight improving according to claim 1, wherein: the selected Chinese herbal raw materials are: the ginkgo leaves: 3-9 parts, the wood butterfly: 6-9 parts, the feather cockscomb seed: 3-15 parts, the goat liver : 30-60 parts, the dream flowers: 3-15 parts, the buddleia: 6-15 parts, the cassia: 6-15 parts, the nux prinsepiae: 3-10 parts, and the aeschynomene: 15-30 parts, the 9 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group C, and the two halves oval capsule are sealed by the paste to obtain the soft capsule products ready for packaging and shipping.

13. The herbal confectionery candy for eyesight improving according to claim 1, wherein: the selected Chinese herbal raw materials are: the ginkgo leaves: 3-9 parts, the wood butterfly: 6-9 parts, the feather cockscomb seed: 3-15 parts, the dream flowers: 3-15 parts, the flos eriocauli: 9-12 parts, the buddleia: 6-15 parts, the cassia: 6-15 parts, the rabbit liver: 30-60 parts, and the aeschynomene: 15-30 parts, the 9 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group C, and the two halves oval capsule are sealed by the paste to obtain the soft capsule products ready for packaging and shipping.

14. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the ghost mesh: 6 parts, the sophora fruit: 5-15 parts, the carrot: 30-120 parts, and the sub shepherd's purse: 10-30 parts, 7 Chinese herbal raw materials are cut into the filaments or pulverized into the granules to improve the decoction extraction efficiency, the carrot is washed and squeezed into juice for using, carrot dregs are cut and extracted by the decoction, and carrot decoction liquid is mixed with the juice; all the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with a candy core tablet made of a human lens and retinal photoreceptor nutrients in a group D, and the two halves oval capsule are sealed by the paste to obtain the soft capsule products ready for packaging and shipping.

15. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the chrysanthemum: 10-15 parts, the glossy privet leaves: 10-15 parts, the carrot: 30-120 parts, the hydrangea wind: 15-30 parts, the 7 Chinese herbal raw materials are cut into the filaments or pulverized into the granules to improve the decoction extraction efficiency, the carrot is washed and squeezed into juice for using, carrot dregs are cut and extracted by the decoction, and carrot decoction liquid is mixed with the juice; all the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group D, and the two halves oval capsule are sealed by the paste to obtain the soft capsule products ready for packaging and shipping.

16. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the senecio: 15-30 parts, the abalone shell: 10-30 parts, the carrot: 30-120 parts, the aeschynomene: 15-30 parts, the 7 Chinese herbal raw materials are cut into the filaments or pulverized into the granules to improve the decoction extraction efficiency, the carrot is washed and squeezed into juice for using, carrot dregs are cut and extracted by the decoction, and carrot decoction liquid is mixed with the juice; all the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group D, and the two halves oval capsule are sealed by the paste to obtain the soft capsule products ready for packaging and shipping.

17. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the dream flowers: 3-15 parts, the buddleia: 6-15 parts, the nux prinsepiae: 3-10 parts, the aeschynomene: 15-30 parts, the 7 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group D, and the two halves oval capsule are sealed by the paste to obtain the soft capsule products ready for packaging and shipping.

18. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the flos eriocauli: 9-12 parts, the cassia: 6-15 parts, the nux prinsepiae: 3-10 parts, the albizia flower: 3-9 parts, the 7 Chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group D, and the two halves oval capsule are sealed by the paste to obtain the soft capsule products ready for packaging and shipping.

19. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the equisetum: 3-10 parts, the abalone shell: 10-30 parts, the nux prinsepiae: 3-10 parts, the sallucidum: 0.9-1.5 parts, the 7 chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group D, and the two halves oval capsule are sealed by the paste to obtain the soft capsule products ready for packaging and shipping.

20. The herbal confectionery candy for eyesight improving according to claim 1, wherein: the selected Chinese herbal raw materials are: the ginkgo leaves: 3-9 parts, the wood butterfly: 6-9 parts, the ligustrum fruit: 6-15 parts, the semen thlaspi: 5-15 parts, the yemingsha: 3-10 parts, the nux prinsepiae: 3-10 parts, the tribulus terrestris: 6-9 parts, the 7 chinese herbal raw materials are cut into the filaments or pulverized into the granules to improve the decoction extraction efficiency, the decoction liquid plus the sugar is concentrated into the paste by the decompressing inner ebullition method, during the cooling and solidification process, the Chinese herbal materials are pressed into the symmetrical two-half oval capsule shell container, then fill the symmetrical two-half oval capsule shell container with the candy core tablet made of the human lens and retinal photoreceptor nutrients in the group D, and the two halves oval capsule are sealed by the paste to obtain the soft capsule products ready for packaging and shipping.
